# EUROPÄISCHE PATENTSCHRIFT

(11) **EP 0 640 324 B1**
(45) Veröffentlichungstag und Bekanntmachung des Hinweises auf die Patenterteilung: **20.05.1998**
(21) Anmeldenummer: 93810616.8
(22) Anmeldetag: 30.08.1993
(51) Int. Cl.: A61F 2/34

(54) **Künstliche Hüftgelenkpfanne**
Artificial acetubular cup
Cupule acétubulaire artificièlle

(43) Veröffentlichungstag der Anmeldung: 01.03.1995
(73) Patentinhaber: Sulzer Orthopädie AG, 6340 Baar (CH)
(72) Erfinder: Maumy, Jean, Dr., F-82000 Montauban (FR); Willi, Roland, CH-8413 Neftenbach (CH)
(74) Vertreter: Heinen, Detlef

(56) Entgegenhaltungen:
- EP-A- 0 253 941
- EP-A- 0 281 984
- EP-A- 0 388 745
- EP-A- 0 445 068
- EP-A- 0 490 616
- EP-A- 0 551 794

## Beschreibung

Die Erfindung bezieht sich auf eine künstliche Hüftgelenkpfanne gemäss dem Oberbegriff von Anspruch 1.

Hüftgelenkpfannen werden oft als Doppelschalen ausgeführt, wobei die Aussenschale eine Verankerung im Knochengewebe erlaubt, und die Innenschale eine sphärische Fläche aufweist, um den Kugelkopf eines Prothesenschaftes aufzunehmen. Aussen- und Innenschale können aus unterschiedlichen Materialien bestehen, um einerseits eine Aussenschale mit einer Gewebefreundlichkeit bezüglich dem Einwachsen zu verwenden und andererseits für die Innenschale Materialien mit guten Eigenschaften bezüglich Verschleiss- und Notlaufverhalten zu verwenden.

Aus der EP 0 313 762 A1 ist eine Hüftgelenkpfanne bekannt, bei der eine Innenschale aus Kunststoff durch eine Schnappverbindung in der Aussenschale verankert ist. Bei diesen Ausführungen zeichnet sich die Aussenschale durch eine hohe Steifigkeit aus, was eine hohe Formbeständigkeit bewirkt. Die Innenschale aus Kunststoff ist abhängig von der Krafteinwirkung des Kugelkopfes elastisch verformbar, und wird dabei durch die steife Aussenschale gestützt. Die steife Aussenschale hat jedoch den Nachteil, dass bei einem unter grossen Druckkräften elastisch nachgebenden Knochen auf der entlasteten Seite Zug- und Schubkräfte zwischen Aussenschale und Knochen auftreten.

Aus der EP 0 445 068 A1 ist eine zweiteilige Hüftgelenkpfanne bekannt, deren Kontur der Aussenschale kleinen Veränderungen des stützenden Knochengewebes folgen kann und dadurch für einen Kraftausgleich zum Knochengewebe sorgt. Beide Schalen sind aus Metall gefertigt, wobei die Wandstärke der Aussenschale sehr dünn ist im Vergleich zur Wandstärke der Innenschale. Die Innenschale hat den Nachteil, dass sie notwendigerweise aus Metall ausgeführt sein muss, um eine hohe Formbeständigkeit der Innenfläche, auf der der Kugelkopf aufliegt, zu gewährleisten. Die elastische Aussenschale hat den Nachteil, dass unmittelbar nach der Implantation das Anwachsen des Knochens in gewissen Fällen schwieriger ist, da während dem Anwachsen Relativbewegungen möglich sein könnten. Weiter kann die elastische Aussenschale nur im Rahmen ihrer elastischen Vorverformung bei der Primärverankerung den späteren einwirkenden Zug- und Schubkräften zwischen Aussenschale und Knochen folgen.

Der Erfindung liegt die Aufgabe zugrunde, eine zweiteilige Hüftgelenkpfanne zu schaffen, die die erwähnten Nachteile beseitigt. Erfindungsgemäss wird diese Aufgabe gelöst gemäss den kennzeichnenden Merkmalen von Anspruch 1. Die Unteransprüche beziehen sich auf weitere, vorteilhafte Ausführungsformen der erfindungsgemässen Vorrichtung.

Die erfindungsgemässe Hüftgelenkpfanne weist verschiedenste Vorteile auf. Die Aussenschale setzt sich aus mehreren, relativ steifen Aussenschalensegmenten zusammen, die durch federnde Bereiche untereinander verbunden sind. Die Aussenschale weist somit Bereiche hoher Formbeständigkeit auf, die untereinander elastisch verbunden sind. Das Aussenschalensegment im Bereich der Hauptbelastungsrichtung ist als Verankerungsbereich ausgeführt, und weist Durchtrittsöffnungen für Knochenschrauben auf, sowie eine Führungs- und Befestigungsbohrung für die Innenschale. Die Führungsbohrung ist bezüglich der gesamten Aussenschale am Pol angeordnet. Die Innenschale der erfindungsgemässen Hüftgelenkpfanne kann aus Metall oder aus Kunststoff gefertigt sein. Im Bereich der Hauptbelastungsrichtung liegt die Aussenschale, das heisst, der Verankerungsbereich, formschlüssig auf der Innenschale auf, was bei Innenschalen aus Kunststoff Spannungsspitzen sowie eine plastische Verformung verhindert. Die übrigen Aussenschalensegmente liegen nicht unmittelbar auf der Innenschale auf, sodass durch die glockenförmige Aufhängung der Innenschale in der Führungsbohrung der Aussenschale die Innenschale insbesondere in radialer Richtung beweglich ist, wobei Dämpfungselemente der Innenschale insbesondere stossförmige Krafteinwirkungen gedämpft an die Aussenschale übertragen. Die elastisch verbundenen Aussenschalensegmente vermögen den Bewegungen des Knochens zu folgen, sodass zum Beispiel an der Aussenschale angewachsenes Knochengewebe nicht abreisst. Besonders vorteilhaft wirkt sich die Verwendung von resorbierbaren Hilfskörpern aus, die, zwischen die Spalten der Aussenschalensegmente eingefügt, der Aussenschale während längerer Zeitdauer eine hohe Formbeständigkeit gewähren. Zwischen Aussenschale und Knochen ergeben sich geringe Relativbewegungen und das Knochengewebe kann gut an die Aussenfläche der Aussenschale anwachsen. Durch den Abbau der resorbierbaren Hilfskörper wird die Steifigkeit der Aussenschale längerfristig nach der Implantation reduziert, sodass die Kontur der Aussenschale den Veränderungen des angewachsenen, stützenden Knochengewebes folgen kann und für den Kraftausgleich zum Knochengewebe gesorgt ist.

Die Innenschale besteht in bekannter Weise aus einem in der Implantat-Technik gebräuchlichen Kunststoff, beispielsweise Polyäthylen, oder aus einem körperfreundlichen Metall. Die Aussenschale besteht aus einem körperfreundlichen Metall, bespielsweise Reintitan oder einer Titanlegierung.

Im folgenden wird die Erfindung anhand von Ausführungsbeispielen beschrieben. Es zeigen:
- Fig. 1a: Eine Untenansicht einer Aussenschale;
- Fig. 1b: ein Schnitt durch die Aussenschale entlang A-A;
- Fig. 1c: eine Aufsicht einer weiteren Aussenschale;
- Fig. 1d: eine Seitenansicht C der Aussenschale;
- Fig. 2a: eine Untenansicht einer Innenschale;
- Fig. 2b: ein Schnitt durch die Innenschale;
- Fig. 3: ein Schnitt durch eine zweiteilige Hüftgelenkpfanne.

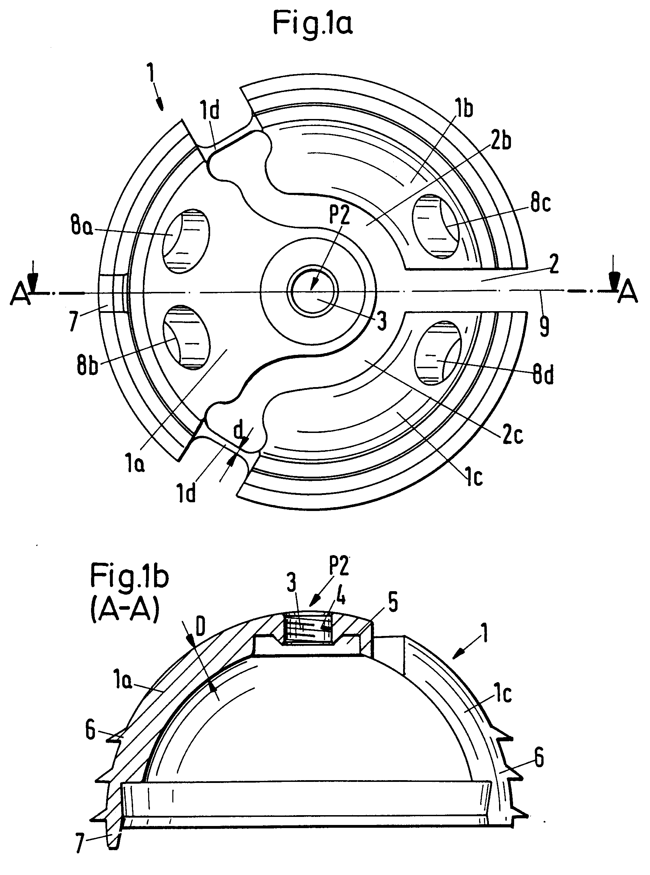

Fig. 1a zeigt eine Untenansicht in die Öffnung einer metallischen Aussenschale 1, die im wesentlichen aus einem Verankerungsbereich 1a sowie zwei über federnde Verbindungselemente 1d mit dem Verankerungsbereich 1a verbundene Aussenschalensegmente 1b und 1c gebildet wird. Der Verankerungsbereich 1a lässt sich mit Verankerungshilfen wie Dornen 6 oder mit Knochenschrauben, die durch die Bohrungen 8a, 8b verlaufen, im Knochengewebe primär verankern. Aus dem Schnitt durch die Aussenschale 1 entlang A-A ist in Fig. 1b die Lage des Poles P2 ersichtlicht. Der Pol P2, ein Bestandteil des Verankerungsbereiches 1a, ist bezüglich der gesamten Aussenschale 1 an deren Pol angeordnet. Im Bereich des Pols P2 ist eine längs einer Montageachse 18 verlaufende Zentrier- oder Führungsbohrung 3 angebracht, die über eine glatte Innenfläche oder ein Gewinde 4 verfügt. Die Zentrierbohrung 3 läuft in eine kegelige, sich nach aussen hin öffnende Ausnehmung 5 aus. Im Bereich des Äquators der Aussenschale 1 sind beidseitig vom Verankerungsbereich 1a ausgehend in Umfangsrichtung je ein Federelement 1d der Breite d angeordnet, das jeweils ein Aussenschalensegment 1b, 1c mit dem Verankerungsbereich 1a verbindet. Die Aussenschalensegmente 1b, 1c können ebenfalls mit Verankerungshilfen wie Dornen 6 oder mit Knochenschrauben, die durch die Bohrungen 8c, 8d verlaufen, im Knochengewebe primär verankerbar sein. Zwischen den Aussenschalensegmenten 1b, 1c und dem Verankerungsbereich 1a bildet sich im vorliegenden Ausführungsbeispiel ein S-förmiger Spalt 2b, 2c. Zwischen den Aussenschalensegmenten 1b, 1c bildet sich ein in Richtung eines Meridiankreises 9 verlaufender radialer Spalt 2. Der Verlauf der Spalten 2, 2b, 2c sowie deren Spaltbreite kann in unterschiedlichster Ausführungsform realisiert werden. Die federnden Eigenschaften zwischen einem Aussenschalensegment 1b, 1c und dem Verankerungsbereich 1a lassen sich durch die Anordnung und die Ausgestaltung des Federelementes 1d in weiten Grenzen variieren. Das dargestellte Federelement 1d weist in radialer Richtung eine relativ geringe Wandstärke d auf, sowie in seiner Ausdehnung parallel zur Montageachse 18 eine im Vergleich zur Wandstärke d vergrösserte Wandstärke d2. Die geringste Steifigkeit des Federelementes 1d ergibt sich somit gemäss Fig. 1a beim Zusammenpressen oder Spreizen der Aussenschale 1 in Umfangrichtung. Entsprechend der Anordnung sowie der Ausgestaltung des Federelementes 1d lassen sich die federnden Eigenschaften zwischen Verankerungsbereich 1a und Aussenschalensegment 1b, 1c in weiten Bereichen variieren.

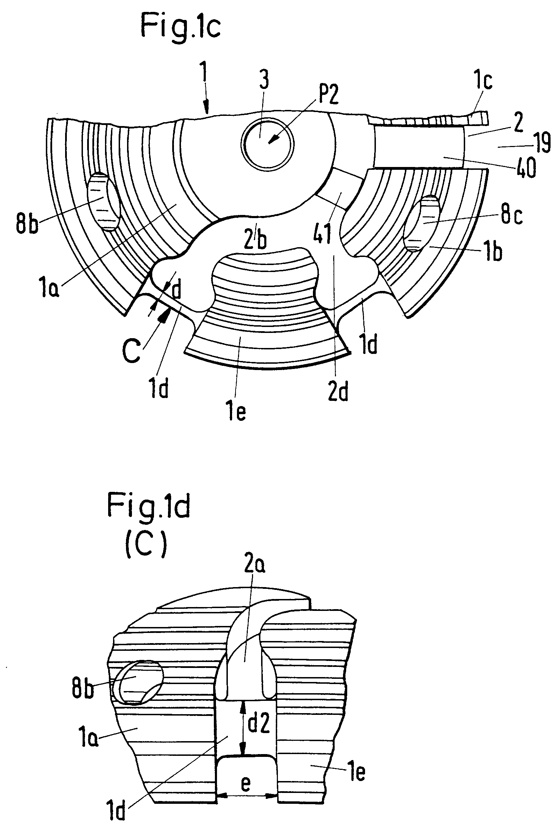

In Fig. 1c ist eine Aufsicht eines weiteren Ausführungsbeispieles einer Aussenschale 1 dargestellt. Der Verankerungsbereich 1a ist gleich ausgestaltet wie in Fig. 1a und weist somit eine Bohrung 8b, einen Pol P2 mit Zentriebohrung 3 und ein in Umfangsrichtung verlaufendes Federelement 1d auf. Dem Federelement 1d folgen in Umfangsrichtung mehrere Aussenschalensegmente 1e, 1b, die durch jeweils mindestens ein Federelement 1d untereinander verbunden sind. Zwischen den Aussenschalensegmenten ergeben sich Spalten 2b, 2d, die nur durch Federelemente 1d überbrückt sind. Die Aussenschalensegmente 1e, 1b können Bohrungen 8c aufweisen, um das Aussenschalensegment mit einer Knochenschraube im Knochen zu verankern. Die Ansicht C der Fig. 1c zeigt in Fig. 1d eine Seitenansicht des Federelementes 1d, das eine Höhe d2 aufweist, und eine Spaltbreite e zwischen dem Verankerungsbereich 1a und dem Aussenschalensegment 1e überbrückt. Der Verlauf des Spaltes zwischen Verankerungsbereich 1a und dem Aussenschalensegment 1e kann auf unterschiedlichste Art ausgestaltet sein, zum Beispiel wie dargestellt derart, dass sich die Spaltbreite nach dem Federelement 1d verjüngt. Ein Spalt 2a, 2d kann sich natürlich in dessen Verlauf auch verbreitern, sowie parallele oder gegenseitig abgewinkelt verlaufende Seitenwände aufweisen. Pro Aussenschalensegment 1e, 1b ist mindestens je ein Federelement 1d notwendig, um zum Verankerungsbereich 1a eine durchgehende Verbindung zu erzielen. Im dargestellten Ausführungsbeispiel sind die Federelemente 1d entlang der Umfangsrichtung der Aussenschale 1 angeordnet. Die Federelemente 1d können jedoch abhängig von der angestrebten Federwirkung auch an anderen Orten angebracht sein, um eine direkte Verbindung zwischen Aussenschalensegment 1e, 1b und Verankerungsbereich 1a zu erreichen oder um Aussenschalensegmente 1e, 1b untereinander zu verbinden. Aussenschalen 1 sind üblicherweise symmetrisch aufgebaut, sodass die in Fig. 1c dargestellte Ebene 19 eine Symmetrieebene bildet, und sich somit zwischen den Aussenschalensegmenten 1b, 1c ein Spalt 2 ergibt.
Zum Einsetzen der Aussenschale 1 in eine Knochenausnehmung können die Aussenschalensegmente 1c, 1b, 1e mit einem Werkzeug derart gefasst und gegen die Montageachse 18 hin vorgespannt werden, dass sich der Aussenumfang der Aussenschale reduziert, dass die Aussenschale somit in die vorbereitete Knochenausnehmung gesetzt werden kann und dass die Aussenschale nach dem Einsetzen gespreizt wird, sodass die eingesetzte Aussenschale die erforderliche, kugelförmige Innenfläche annimmt. Die Steifigkeit der Aussenschale 1 lässt sich zum Beispiel beim Einsetzen erhöhen, wenn zwischen die Aussenschalensegmente 1b, 1c und/oder zwischen den Aussenschalensegmenten 1b, 1e und dem Verankerungsbereich 1a entfernbare Körper 40, 41 eingefügt werden. Die Aussenschale 1 weist somit beim Einschlagen in die Knochenausnehmung äusserst steife Eigenschaften auf. Beim Einschlagen der Aussenschale 1 werden die Ränder der Knochenausnehmung durch die Verankerungshilfen 6 zusätzlich verletzt, was jedoch dem Anwachsen des Knochens an die äussere Oberfläche der Aussenschale 1 förderlich sein kann. Es kann sich als vorteilhaft erweisen, wenn die Aussenschale 1 während den ersten Monaten nach der Implantation über sehr steife Eigenschaften verfügt, damit der Knochen problemlos an der äusseren Oberfläche der Aussenschale 1 anwachsen kann. Dies lässt sich erreichen, wenn vor oder nach dem Einschlagen der Aussenschale 1 in die Knochenausnehmung Körper 40, 41 aus resorbierbarem Material in die Spalten 2, 2b, 2d eingefügt werden. Die resorbierbaren Körper 40, 41 verleihen der Aussenschale 1 anfänglich steife Eigenschaften, wobei die Körper 40, 41 längerfristig abgebaut werden und die stützende Wirkung somit verlieren.

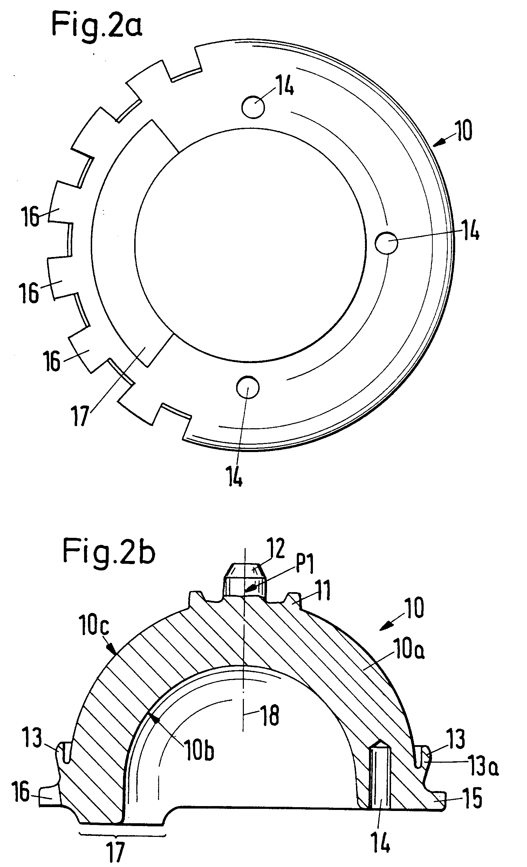

Fig. 2b zeigt einen Schnitt durch eine Innenschale 10, an deren Pol P1 ein Zentrierzapfen 12 sowie ein bezüglich dem Zentrierzapfen 12 konzentrisch verlaufender, ringförmiger, örtlich verformbarer Steg 11 angeordnet ist. Am Äquator weist die Innenschale 10 einen vorstehenden Kranz 15 auf, der teilweise durchbrochen ist, sodass sich mehrere, radial nach aussen stehende Vorsprünge 16 ergeben. Anschliessend an den Kranz 15 weist die Aussenfläche 10c ein elastisches Abstützelement 13 auf, das ringförmig um die Innenschale 10 verläuft und im vorliegenden Ausführungsbeispiel aus der Wand 10a der Innenschale 10 gearbeitet ist, indem der vorstehende Bereich 13 der Innenschale 10 hinterstochen wurde, sodass eine federnde Lippe 13a entstand, die ein Bestandteil der Innenschale 10 bildet. Die Innenfläche 10b der Innenschale 10 ist sphärisch ausgestaltet, um einen Kugelkopf eines Gelenkschaftes abzustützen. Fig. 2a zeigt eine Untenansicht der Innenschale 10. Die drei Bohrungen 14 dienen dazu, die Innenschale 10 mit einer Hilfshalterung zu fassen und in die Aussenschale 1 einzufügen. Weiter sind die radial nach aussen stehenden Vorsprünge 16 ersichtlich, sowie eine Überhöhung 17, die auf der Seite der grössten Krafteinleitung angeordnet ist.

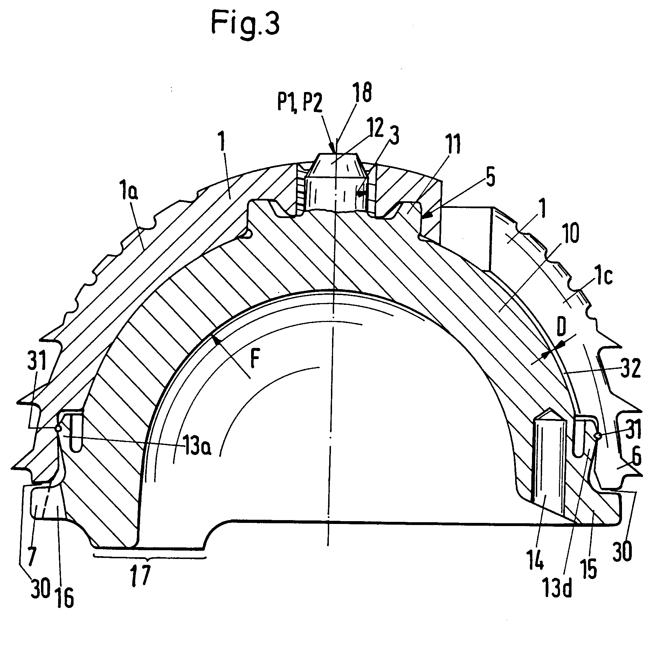

Fig. 3 zeigt eine zweiteilige Hüftgelenkpfanne in zusammengesetztem Zustand. Nachdem die Aussenschale 1 im Knochen verankert ist, wird die Innenschale 10 mit einer Hilfshalterung, die in die Bohrungen 14 eingreift, in Richtung der Montageachse 18 in die Aussenschale 1 eingeführt. Der Zentrierzapfen 12 der Innenschale 10 und die Führungsbohrung 3 der Aussenschale 1 dienen zum Zentrieren und Führen der Innenschale 10 beim Einpressen in die Aussenschale. Der Zentrierzapfen 12 bildet mit der Führungsbohrung 3 eine form- oder kraftschlüssige Verbindung. Beim Zusammenfügen kommt gleichzeitig der ringförmige Steg 11 der Innenschale 10 in die konusförmige Ausnehmung 5 der Aussenschale 1 zu liegen und bildet ebenfalls eine form- oder kraftschlüssige Verbindung, üblicherweise ein Schnappverschluss. Im Bereich des Äquators kommt die federnde Lippe 13a der elastischen Abstützung 13 auf die Innenfläche der Aussenschale 1 zu liegen, wobei sich der Innendurchmesser der Aussenschale 1 in diesem Bereich gegen den Pol P1 hin leicht erweitert, sodass sich im Bereich des Äquators ein Schnappverschluss zwischen Innenschale 10 und Aussenschale 1 ergibt. Der Verankerungsbereich 1a der Aussenschale 1 weist eine in Richtung der Montageachse 18 vorstehende Nocke 7 auf.

Die gegenseitige Lage von Innenschale 10 und Aussenschale 1 in ihrer äquatorialen Ausrichtung ist unmittelbar vor dem Einsetzen der Innenschale 10 bestimmbar. Die Innenschale 10 weist radial nach aussen stehende Vorsprünge 16 auf. Die Innenschale 10 ist derart in die Aussenschale 1 einzuführen, dass die Nocke 7 zwischen zwei Vorsprünge 16 zu liegen kommt, sodass Innen- und Aussenschale im eingepressen Zustand bezüglich einer gegenseitigen Bewegung in äquatorialer Richtung fixiert sind.

Innen- wie Aussenschale sind an deren gemeinsamem Pol P1, P2 fest miteinander verbunden, wogegen die Innenschale 10 sich im Bereich des Äquators über den gesamten Umfang durch die federnde Lippe 13a elastisch an der Aussenschale 1 abstützt, und somit eine Auflage 31 bildet, derart, dass die Innenschale 10 in einer senkrecht zur Montageachse 18 verlaufenden Richtung pendelnde Bewegungen ausführen kann.

Die zweiteilige Hüftgelenkpfanne weist bezüglich dem Kräftespiel ein statisches sowie ein dymanisches Verhalten auf. Im statischen Fall, der in Fig. 3 dargestellt ist, liegt die Innenfläche des Verankerungsbereiches 1a auf der Aussenfläche der Innenschale 10 auf. Die ungefähre Richtung einer von einem Kugelkopf auf eine Hüftgelenkpfanne bewirkten Reaktionskraft F zeigt, wie in Fig. 3 dargestellt, in Richtung des Verankerungsbereiches 1a. Somit übt der Verankerungsbereich 1a im Bereich der grössten statischen Belastung eine stützende Wirkung auf die Innenschale 10 aus. Dadurch ist es möglich, die Innenschale 10 auch aus Kunststoff zu fertigen, da die stützende Wirkung ein Fliessen des Kunststoffes verhindert. Im statischen Fall weisen die beweglichen Bereiche 1b, 1c von der Aussenfläche der Innenschale 10 einen Abstand D auf. Im dynamischen Fall werden vom Kugelkopf stossartige Reaktionskräfte in unterschiedlichster Richtung auf die Innenschale 10 übertragen. Die Innenschale 10 ist in Richtung der beweglichen Bereiche 1b, 1c nur leicht abgestützt, sodass sich die Innenschale 10 in diese Richtung auszuweichen versucht, und sich somit eine pendelnde Bewegung der Innenschale 10 um den Pol P1, P2 ergibt. Die Druckspitzen werden auf die beweglichen Bereiche 1b, 1c der Aussenschale 1 übertragen, welche diese gedämpft in das Knochengewebe übertragen. Dabei folgen die beweglichen Bereiche 1b, 1c der Aussenschale 1 den Bewegungen des Knochengewebes, sodass sich das angewachsene Knochengewebe auch bei hohen Druckspitzen nicht von der Aussenfläche der beweglichen Bereiche 1b, 1c löst. Der Kranz 15 sowie der Vorsprung 16 der Innenschale 10 weist zur Aussenschale 1 ein Spiel 30 auf, das nur bei hohen Druckspitzen aufgehoben wird, und die relative Bewegung zwischen Innen- und Aussenschale begrenzt.
Die Aussenschale 1 passt sich sowohl bei einwirkenden Kräften als auch bei kleinen Veränderungen des Knochengewebes elastisch an, sodass die Zug- und Schubkräfte zwischen Aussenschale 1 und Knochengewebe relativ klein bleiben.

## Patentansprüche

1. Künstliche Hüftgelenkpfanne bestehend aus einer metallischen Aussenschale (1) mit Verankerungshilfen (6, 8a, 8b) und einer Innenschale (10) zur Aufnahme eines Gelenkkugelkopfes, wobei die Innenschale (10) an ihrer Aussenfläche in Umfangsrichtung ein ringförmiges Dämpfungselement (13) und an ihrem Pol (P1) einen Zentrierzapfen (12) aufweist, und wobei die Aussenschale (1) an deren Pol (P2) eine zur Rotations-Symmetrieachse der Aussenschale (1) konzentrische Führung (3) zur Aufnahme des Zentrierzapfens (12) aufweist, dadurch gekennzeichnet,
dass die Aussenschale (1) einen Verankerungsbereich (1a) aufweist sowie in Umfangsrichtung Federbereiche (1d) geringer Wandstärke (d) alternierend mit Aussenschalensegmenten (1b, 1c, 1e) grösserer Wandstärke (D),
dass der Verankerungsbereich (1a) Verankerungshilfen (6, 8a, 8b) sowie die Führung (3) aufweist, und dass die Aussenschale (1) auf der dem Verankerungsbereich (1a) gegenüberliegenden Seite einen zum Äquator hin geöffneten Spalt (2) aufweist, der entlang eines durch den Pol (P2) verlaufenden Meridiankreises (9) verläuft.

2. Vorrichtung nach Anspruch 1, dadurch gekennzeichnet, dass die Innenschale (10) aus Metall besteht.

3. Vorrichtung nach Anspruch 1, dadurch gekennzeichnet, dass die Innenschale (10) aus Kunststoff besteht.

4. Vorrichtung nach einem der Ansprüche 1 bis 3, dadurch gekennzeichnet, dass das Dämpfungselement (13) aus Polyäthylen besteht.

5. Vorrichtung nach Anspruch 3, dadurch gekennzeichnet, dass das Dämpfungselement (13) Bestandteil der Innenschale (10) ist, indem die Aussenfläche der Innenschale (10) äquatornah in Umfangsrichtung hinterstochen ist, und dadurch eine federnde Lippe (13a) aufweist.

6. Vorrichtung nach einem der Ansprüche 1 bis 5, dadurch gekennzeichnet, dass im zusammengesetzten, unbelasteten Zustand der Verankerungsbereich (1a) der Aussenschale (1) die Innenschale (10) grossflächig berührt, wogegen zwischen den Aussenschalensegmenten (1b, 1c, 1e) und der Innenschale (10) mit Ausnahme des Dämpfungselementes (13) ein Spiel (32) besteht.

7. Vorrichtung nach einem der Ansprüche 1 bis 6, dadurch gekennzeichnet, dass das Dämpfungselement (13) sich radial an einer Ausnehmung der Aussenschale (1) abstützt und dabei ein Schnappverschluss bildet.

8. Vorrichtung nach einem der Ansprüche 1 bis 7, dadurch gekennzeichnet, dass mindestens zwei Aussenschalensegmente (1b, 1c) Verankerungshilfen (8c, 8d) aufweisen.

9. Vorrichtung nach einem der Ansprüche 1 bis 8, dadurch gekennzeichnet, dass die Aussenschale (1) mehrere Spalte (2, 2b, 2c, 2d) aufweist, und dass mindestens in einem Spalt (2, 2b, 2c, 2d) ein resorbierbarer Körper (40, 41) einsetzbar ist, um der Aussenschale (1) eine erhöhte Primärstabilität zu verleihen.

10. Vorrichtung nach Anspruch 9, dadurch gekennzeichnet, dass die resorbierbaren Körper (40, 41) aus Polylaktat bestehen.

## Claims

1. An artificial acetubular cup comprising of a metallic outer shell (1) having anchorage aids (6,8a,8b) and an inner shell (10) to incorporate a ball-joint head, whereby inner shell (10) has an annular-shaped damping element (13) on its outer surface in the circumferential direction and has a centring pin (12) on its pole (P1), and where outer shell (1) has a guide (3), concentric to the rotation symmetry axis, for incorporating the centring pin (12), characterised in that outer shell (1) has an anchorage area (1a), likewise spring areas (1d) in the circumferential direction, having a small wall thickness (d), alternating with outer shell segments (1b,1c,1e) of greater wall thickness (D), that anchorage area (1a) has anchorage aids (6,8a,8b) likewise the guide (3), and that outer shell (1) has a gap (2) on the side opposite the anchorage area (1a) opening to the equator, which extends along a meridian circle (9) through pole (P2).

2. A device according to claim 1, characterised in that inner shell (10) is comprised of metal.

3. A device according to claim 1, characterised in that inner shell (10) is comprised of synthetic material.

4. A device according to claims 1 to 3, characterised in that damping element (13) is comprised of polyethylene.

5. A device according to claim 3, characterised in that the damping element (13) is a component of inner shell (10), in which the outer surface of inner shell (10) close to the equator is recessed in the circumferential direction, and thereby has a springy lip (13a).

6. A device according to any of claims 1 to 5, characterised in that anchorage area (1a) of outer shell (1) is in extensive contact with inner shell (10) in the assembled, non-loaded state, against which a clearance (32) is present between outer shell segments (1b,1c,1e) and inner shell (10) with the exception of damping element (13).

7. A device according to any of claims 1 to 6, characterised in that damping element (13) is radially supported on a recess of outer shell (1), and thus forms a snap fastening.

8. A device according to any of claims 1 to 7, characterised in that at least two outer shell segments (1b,1c) have anchorage aids (8c,8d).

9. A device according to any of claims 1 to 8, characterised in that outer shell (1) has a plurality of gaps (2,2b,2c,2d) and that a re-absorbable body (40,41) is insertable in at least one gap (2,2b,2c,2d), so as to lend an increased primary stability to outer shell (1).

10. A device according to claim 9, characterised in that the reabsorbable bodies (40,41) are comprised of polylactate.

## Revendications

1. Cupule acétabulaire artificielle constituée d'une coque extérieure métallique (1) avec des aides à l'ancrage (6, 8a, 8b) et une coque intérieure (10) pour recevoir une tête sphérique articulaire, la coque intérieure (10) présentant à sa surface extérieure dans la direction de pourtour un élément d'amortissement annulaire (13) et à son pôle (P1) un pivot de centrage (12), et où la coque extérieure (1) présente à son pôle (P2) un guidage (3) concentrique à l'axe symétrique de rotation de la coque extérieure (1) pour la réception du pivot de centrage (12), caractérisée en ce que la coque extérieure (1) présente une zone d'ancrage (1a) ainsi que dans la direction de pourtour des zones élastiques (1d) d'une épaisseur de paroi plus réduite (d) alternant avec des segments de coque extérieure (1b, 1c, 1e) d'une plus grande épaisseur de paroi (d),
en ce que la zone d'ancrage (1a) présente des aides à l'ancrage (6, 8a, 8b) ainsi que le guidage (3), et en ce que la coque extérieure (1) présente sur le côté opposé à la zone d'ancrage (1a) une fente (2) ouverte vers l'équateur qui s'étend le long d'un cercle de méridienne (9) s'étendent à travers le pôle (P2).

2. Dispositif selon la revendication 1, caractérisé en ce que la coque intérieure (10) est réalisée en métal.

3. Dispositif selon la revendication 1, caractérisé en ce que la coque intérieure (10) est réalisée en matière synthétique.

4. Dispositif selon l'une des revendications 1 à 3, caractérisé en ce que l'élément d'amortissement (13) est réalisé en polyéthylène.

5. Dispositif selon la revendication 3, caractérisé en ce que l'élément d'amortissement (13) fait partie de la coque intérieure (10) en ce que la surface extérieure de la coque intérieure (10) est dépouillée au tour à proximité de l'équateur dans la direction de pourtour et présente de ce fait une lèvre élastique (13a).

6. Dispositif selon l'une des revendications 1 à 5, caractérisé en ce qu'à l'état assemblé, non chargé, la zone d'ancrage (1a) de la coque extérieure (1) vient en contact sur une grande surface avec la coque intérieure (10), où il existe par contre entre les segments de coque extérieure (1b, 1c, 1e) et la coque intérieure (10) à l'exception de l'élément d'amortissement (13), un jeu (32).

7. Dispositif selon l'une des revendications 1 à 6, caractérisé en ce que l'élément d'amortisement (13) prend appui radialement à un évidement de la coque extérieurs (1) et forme ainsi un assemblage par enclenchement.

8. Dispositif selon l'une des revendications 1 à 7, caractérisé en ce qu'au moins deux segments de coque extérieure (1b, 1c) présentent des aides à l'ancrage (8c, 8d).

9. Dispositif selon l'une des revendications 1 à 8, caractérisé en ce que la coque extérieure (1) présente plusieurs fentes (2, 2b, 2c, 2d) et que dans au moins une fente (2, 2b, 2c, 2d), un corps résorbable (40, 41) peut être placé pour conférer à la coque extérieure (1) une plus grande stabilité primaire.

10. Dispositif selon la revendication 9, caractérisé en ce que les corps résorbables (40, 41) sont réalisés en polylactate.
